# EUROPÄISCHE PATENTANMELDUNG

(11) **EP 1 340 976 A1**
(43) Veröffentlichungstag der Anmeldung: **03.09.2003**
(21) Anmeldenummer: 02102817.0
(22) Anmeldetag: 19.12.2002
(51) Int. Cl.: G01N 33/30

(54) **Ölqualitätsmesseinrichtung**

(30) Priorität: 27.02.2002 DE 10208600
(71) Anmelder: FILTERWERK MANN + HUMMEL GMBH, 71638 Ludwigsburg (DE)
(72) Erfinder: Beylich, Markus, 71636 Ludwigsburg (DE); Franz, Andreas, 70806 Kornwestheim (DE)
(74) Vertreter: Voth, Gerhard

(57) **Zusammenfassung**

Die Erfindung betrifft eine Ölqualitätsmeßeinrichtung für einen Ölkreislauf (1). Sie umfaßt einen Ölzustandssensor (2) und eine Auswerteelektronik (3). Dabei ist der Ölzustandssensor (2) als kapazitiver oder impedanzspektroskopischer Sensor (4) ausgebildet und derart mit einem Filterelement (14) verbunden, daß bei einem Auswechseln des Filterelementes (14) zwangsweise auch der Ölzustandssensor (2) ausgewechselt wird.

## Beschreibung

Die Erfindung betrifft eine Ölqualitätsmeßeinrichtung für einen Ölkreislauf.

Der zuverlässige Betrieb eines Ölkreislaufes beispielsweise zur Schmierung einer Brennkraftmaschine in einem Kraftfahrzeug oder einer Hydraulikanlage erfordert eine gute Ölqualität. Im Laufe des Betriebes eines Ölkreislaufes kann sich die Ölqualität beispielsweise durch Verschmutzung, Zersetzung, Wasseraufnahme oder dgl. verschlechtern. Bei Schmierölkreisläufen von Verbrennungsmotoren gelangen Rußpartikel, Metallabrieb und u. U. auch Kraftstoff unerwünscht in das Öl und vermindern dessen Schmierfähigkeit.

Zur Aufrechterhaltung einer bestimmten Ölqualität über einen vorgegebenen Zyklus ist im Ölkreislauf ein Ölfilter angeordnet, der auftretende Verunreinigungen aus dem Öl herausfiltern soll. Insbesondere beim Betrieb von Dieselmotoren können auch korrosive Säuren im Öl auftreten, zu deren Neutralisation entsprechende metallische Werkstoffe im Ölfilter angeordnet sind. Ungünstige Betriebsparameter wie die häufige Durchführung eines Kaltstartes können zu einer frühzeitigen Verschlechterung der Ölqualität bereits innerhalb des vorgegebenen Ölwechselintervalls führen. In der Folge kann ein erhöhter Verschleiß oder gar eine Schädigung der Hydraulikanlage bzw. des Antriebsmotors auftreten.

Zur Vermeidung dieses Problems ist beispielweise aus der US 4,791,374 ein Ölkreislauf bekannt, in dem ein Zustandssensor zur Messung der Ölqualität vorgesehen ist. Bei entsprechender Abweichung des Meßparameters vom Sollbereich kann eine Verschlechterung der Ölqualität frühzeitig erkannt und ein Ölwechsel herbeigeführt werden. An die Qualität und insbesondere die Lebensdauer des Ölzustandssensors sind hohe Anforderungen gestellt, was die Meßeinrichtung teuer und u. U. unwirtschaftlich macht.

Der Erfindung liegt die Aufgabe zugrunde, eine zuverlässige Ölqualitätsmeßeinrichtung für einen Ölkreislauf mit verbesserter Wirtschaftlichkeit bereitzustellen.

Die Aufgabe wird durch eine Ölqualitätsmeßeinrichtung mit den Merkmalen des Anspruchs 1 gelöst.

Dazu wird eine Ölqualitätsmeßeinrichtung für einen Ölkreislauf mit einem Ölzustandssensor und einer Auswerteelektronik vorgeschlagen, wobei der Ölzustandssensor als kapazitiver oder impedanzspektroskopischer Sensor ausgebildet ist. Dieser Sensor ist in einem Ölfilter des Ölkreislaufs angeordnet und zwangsweise als Einheit gemeinsam mit dem Filterelement austauschbar. Die Auswerteelektronik ist getrennt vom Ölfilter ortsfest an einem Gehäuse eines Bauteils des Ölkreislaufs gehalten. Durch die Anordnung eines solchen Sensors zusammen mit einer Auswerteelektronik ist zuverlässig eine Vielzahl von Parametern zur Bestimmung der Ölqualität meßbar. Der Sensor kann dabei ohne Beeinträchtigung seiner Zuverlässigkeit einfach und kostengünstig aufgebaut sein, wobei sein regelmäßiger Austausch gemeinsam mit dem Filterelement die Zuverlässigkeit der Meßeinrichtung insgesamt über eine lange Lebensdauer sicherstellt. Die außerhalb des Ölfilters ortsfest am Ölkreislauf angeordnete Auswerteelektronik ist vor dem Einfluß des Öls geschützt und kann mit geringem Aufwand auf eine lange Lebensdauer über mehrere Ölwechselintervalle oder gar bis zur gesamten Systemlebensdauer ausgelegt werden.

Insbesondere bei Auslegung des Ölzustandssensors und der Auswerteelektronik zur Messung mittels der Impedanzspektroskopie ergibt sich bei geringem Aufwand eine hohe Systemzuverlässigkeit. Bei der Impedanzspektroskopie werden die Elektrizitätskonstanten, der Verlustwinkel und die Leitfähigkeit des Öls in Abhängigkeit einer am kapazitiven Sensor anliegenden Meßspannungsfrequenz bestimmt. Das zu messende Öl mit seiner Vielzahl von u. U. schädlichen Bestandteilen bildet dabei im meßtechnischen Sinne einen komplexen Widerstand. Dessen Impedanzspektrum kann hinsichtlich Realteil und Imaginärteil in der beschriebenen Weise als Funktion der Meßfrequenz bestimmt werden. Jeder Schadstoffanteil im Öl weist eine eigene spezifische Einzelleitfähigkeit auf, die im gemessenen Impedanzspektrum identifizierbar ist. Das zu prüfende Öl kann mit einer einzigen Meßeinrichtung und insbesondere mit einem einzigen, einfach aufgebauten kapazitiven Sensor im Ölfilter hinsichtlich des Gehaltes einer Vielzahl von Schadstoffen geprüft werden. Durch Überwachung der Ölqualität sind die im Ölkreislauf geschmierten bzw. bewegten Teile zuverlässig geschützt. Durch Überwachung der Ölinhaltsstoffe ist auch ein Schutz für das Filterelement gegeben, da ein die Filterelementstandzeit reduzierender Säureanteil im Öl rechtzeitig erkannt werden kann.

Durch die Integration des Sensors in den Ölfilter in Verbindung mit der gemeinsamen Austauschbarkeit ergibt sich auch ein Schutz des Sensorelementes selbst vor korrosiven Säuren im Öl, beispielweise bei Dieselmotoren.

In einer zweckmäßigen Weiterbildung ist ein Filtergehäuse in einen Filterhalter einschraubbar, wobei die elektrische Kontaktierung zwischen dem Ölzustandssensor und der Auswertelektronik über Ringkontakte erfolgt. Eine flächige und zuverlässige Kontaktierung ist unabhängig von der Winkellage des Filterelementes gegeben. Alternativ oder in Kombination kann ein Steckverbinder vorgesehen sein, der insbesondere außerhalb des Ölfiltergehäuses liegt und damit vor korrosiven Einflüssen geschützt ist. Durch Anordnung der Auswerteelektronik direkt am Filterhalter des Ölfilters sind kurze elektrische Wege zwischen dem Sensor und der Auswerteelektronik gegeben, was zur Steigerung der Meßgenauigkeit beiträgt. Die Auswerteelektronik ist dabei zweckmäßig in einem separaten, am Filterhalter befestigten Gehäuse angeordnet, wodurch im Schadensfalle eine leichte Auswechselbarkeit gegeben ist. Auch können modulartig Ölfilter gleicher Bauart wahlweise mit und ohne Ölqualitätsmeßeinrichtung gebaut und ausgeliefert werden, wobei auch die Möglichkeit einer Nachrüstung gegeben ist.

Zur Steigerung der Meßgenauigkeit hat sich eine Anordnung des Ölzustandssensors auf der Reinölseite des Ölfilters als zweckmäßig herausgestellt. Die Fremdstoffkonzentration im Öl ist an dieser Stelle vergleichsweise gering und relativ frei von aufgewirbelten Partikeln, wodurch die Bildung eines klar definierten Meßergebnisses unterstützt ist.

In vorteilhafter Weiterbildung ist der Ölzustandssensor in einer Endscheibe des Filterelementes integriert. Die Endscheibe ist vom zu prüfenden Öl gut umströmt und bietet damit einen geeigneten Ort für eine präzise Messung. Als separates Einzelteil gefertigt, vermeidet sie konstruktive Änderungen an den übrigen Bauteilen des Ölfilters. Zur Steigerung der Präzision des Meßergebnisses hat sich eine Ausbildung des Sensors mit möglichst großer geometrischer Ausdehnung bzw. Meßlänge als zweckmäßig herausgestellt. Dazu ist beispielsweise die spiralförmige oder mäanderförmige Anordnung eines elektrisch leitenden Meßdrahtes, Blechstreifens oder dgl. zweckmäßig, wodurch eine insgesamt große Meßlänge auf kleinem Bauraum untergebracht werden kann.

In einer vorteilhaften Variante, bei der der Ölfilter einen jeweils etwa zylindrischen Stützkörper und ein den Stützkörper umschließendes Filterelement umfaßt, ist der Ölzustandssensor als zylindrischer Hohlkörper ausgebildet und zwischen dem Stützkörper und dem Filterelement angeordnet. Die mechanische Belastung des Zustandssensors ist wegen der sich ergebenden allseitigen Abstützung gering. Gleichzeitig ergibt sich eine gute Umströmung mit Öl, was zur Steigerung der Meßgenauigkeit beiträgt. In vorteilhafter Weiterbildung dieser Variante ist der Stützkörper des Ölfilters selbst als Ölzustandssensor ausgebildet, was zur Reduzierung der Teilezahl und der Systemkomplexität beiträgt.

Insbesondere bei Ölfiltern, bei denen nur das Filterelement als Einzelteil ausgewechselt wird, kann es zweckmäßig sein, den Ölzustandssensor fest an diesem Filterelement aufzubringen. Dadurch ist sichergestellt, daß in den vorgeschriebenen Filterwechselintervallen auch der Sensor mit gewechselt wird, womit ein unzulässiger Betrieb des Sensors über seine konstruktiv vorgegebene Lebensdauer hinaus vermieden wird.

Ausführungsbeispiele der Erfindung werden im Folgenden anhand der Zeichnung näher beschrieben. Es zeigen:
- Fig. 1: in einer Schnittdarstellung einen Ölfilter eines angedeuteten Ölkreislaufes mit einem als Ölzustandssensor ausgebildeten Stützgitter;
- Fig. 2: eine Variante der Anordnung nach Fig. 1 mit einem als zylindrischer Hohlkörper ausgebildeten und zwischen dem Stützkörper und dem Filterelement liegenden Ölzustandssensor;
- Fig. 3: eine Schnittdarstellung durch eine weitere Ölfilteranordnung mit einem drahtförmigen, fest auf dem Filterelement aufgebrachten Ölzustandssensor;
- Fig. 4: in einer Prinzipdarstellung Einzelheiten eines zu einer Wendel aufgewickelten Drahtes zur Bildung eines Ölzustandssensors;
- Fig. 5: eine schematische Darstellung eines mäanderförmig auf eine Endscheibe des Filterelementes aufgebrachten Ölzustandssensors;
- Fig. 6: eine Variante der Anordnung nach Fig. 5 mit einem spiralförmig verlaufenden Ölzustandssensor.

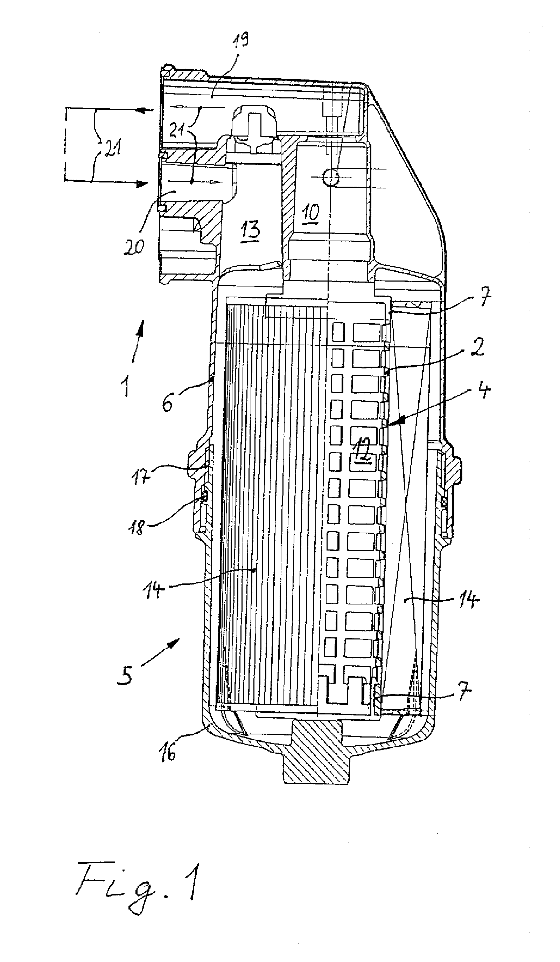

Fig. 1 zeigt in einer Schnittdarstellung einen Ölfilter 5 eines schematisch angedeuteten Ölkreislaufes 1, am Beispiel eines Schmierölkreislaufs für einen LKW-Dieselmotor. Vergleichbare Anordnungen können auch für Schmierölkreisläufe in PKWs, für Getriebeöl- sowie Hydraulikölkreisläufe vorgesehen sein.

Im Ölkreislauf 1 ist ein Ölfilter 5 vorgesehen, der im gezeigten Ausführungsbeispiel ein in einen Filterhalter 6 eingeschraubtes Filtergehäuse (16) aufweist. Im Filtergehäuse 16 ist ein zylindrischer, gitterförmiger Stützkörper 12 sowie ein ebenfalls zylindrisches, den Stützkörper 12 umschließendes Filterelement 14 angeordnet. Der Stützkörper 12 und das Filterelement 14 sind in axialer Richtung zwischen einer Stirnwand des Filtergehäuses 16 und dem Filterhalter 6 gehalten. Das Filtergehäuse 16 ist mittels eines Gewindes 17 in den Filterhalter 6 einschraubbar und über einen umlaufenden O-Ring 18 gegen den Filterhalter 6 abgedichtet.

Die Strömungsrichtung des Öles im Ölkreislauf 1 ist durch die Pfeile 21 angedeutet, demnach das Öl über einen Zulauf 20 zu einer Zuströmseite 13 des Ölfilters 5 geführt wird. Im Ölfilter 5 durchströmt das Öl das Filterelement 14 radial von außen nach innen, wobei das zylindrische Filterelement 14 der entstehenden, radial nach innen wirkenden Druckdifferenz durch die Stützwirkung des Stützkörpers 12 standhält. Radial innenseitig des Filterelementes 14 liegt die Reinölseite 10 des Ölfilters 5, mit der ein Ablauf 19 im Filterhalter 6 strömungsleitend zur Abführung des gefilterten Öls verbunden ist.

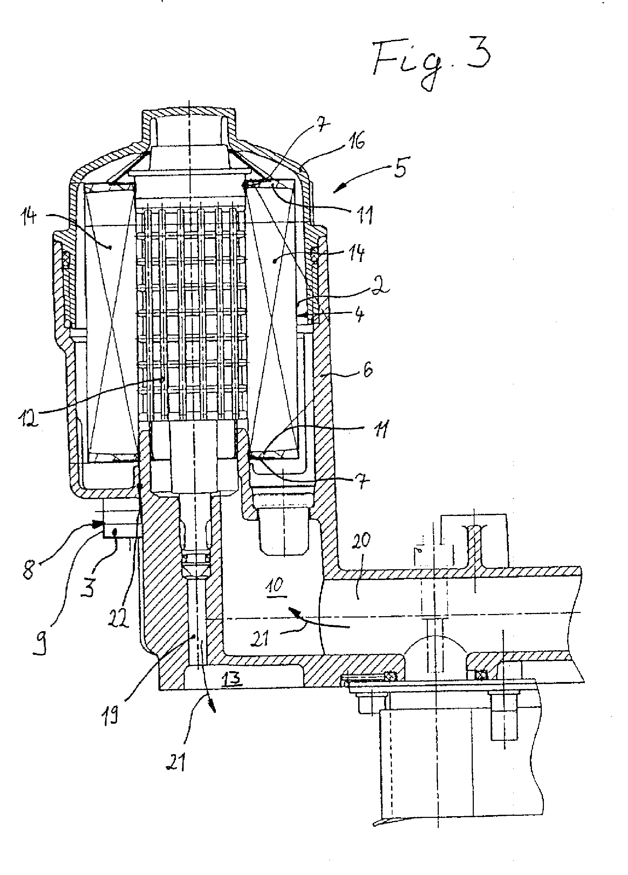

Im Ausführungsbeispiel nach Fig. 1 ist der Stützkörper 12 als ein Ölzustandssensor 2 in Form eines kapazitiven Sensors 4 ausgebildet und liegt damit auf der Reinölseite 10 des Ölfilters 5. Zur elektrischen Kontaktierung des Sensors 2, 4 ist im Bereich der beiden Stirnseiten des Stützkörpers 12 je ein umlaufender Ringkontakt 7 zur Verbindung mit einer im Zusammenhang mit Fig. 3 näher beschriebenen Auswerteelektronik 3 vorgesehen.

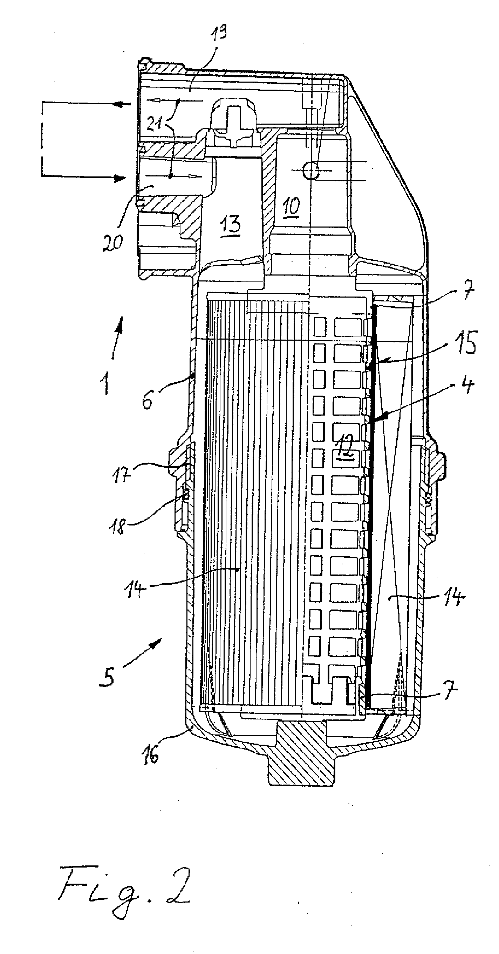

Fig. 2 zeigt eine Variante der Anordnung nach Fig. 1, bei der der kapazitive Sensor 4 als zylindrischer Hohlkörper 15 ausgebildet und zwischen dem Stützkörper 12 und dem Filterelement 14 angeordnet ist. In den übrigen Merkmalen und Bezugszeichen stimmt die Anordnung der Fig. 2 mit der nach Fig. 1 überein. Der in den Fig. 1 und 2 gezeigte kapazitive Sensor 4 kann aus gestanztem oder geätztem Blech, aus gewickeltem oder geflochtenem oder anderweitig geformten Draht, als geätzte Platine oder dgl. ausgebildet sein. Der Sensor 4 ist dabei aus einem elektrisch leitfähigem Material, wobei eine hohe Korrosionsbeständigkeit zweckmäßig ist. Als Materialien haben sich Edelstahl, Titan bzw. Kohlenstoffasern als zweckmäßig herausgestellt.

Fig. 3 zeigt eine weitere Anordnung eines Filters 5 mit einem zylindrischen Stützkörper 12 und einem ebenfalls zylindrischen Filterelement 14, die in einem Filtergehäuse 16 und einem Filterhalter 6 gehalten sind. In axialer Richtung ist beidseitig des Filterelementes 14 je eine Endscheibe 11 vorgesehen. Der Ölzustandssensor 2 ist als kapazitiver Sensor 4 ausgebildet und radial außenseitig auf den austauschbaren Filterkörper 14 fest aufgebracht.

Auf der Außenseite des Filterhalters 6 ist eine Auswerteelektronik 3 in einem separaten Gehäuse 9 angeordnet und ortsfest am Filterhalter 6 gehalten. Zur elektrischen Kontaktierung des Sensors 2, 4 mit der Auswerteelektronik 3 ist eine Kombination aus Ringkontakten 7, einer den Filterhalter 6 von außen nach innen durchgreifenden Leitung 21 sowie eines Steckverbinders 8 vorgesehen. Die Auswerteelektronik 3 im separaten Gehäuse 9 ist bedarfsweise durch Lösen des Steckverbinders 8 austauschbar. Es kann auch eine Anordnung zweckmäßig sein, bei der die Auswerteelektronik 3 im Filterhalter 6 integriert ist. Bei den in den Fig. 1 bis 3 gezeigten Ausführungsbeispielen ist der Ölfilter 5 bzw. der Sensor 2, 4 zum gemeinsamen Austausch mit dem Filterelement 14 vorgesehen. Es kann auch eine gemeinsame Austauschbarkeit mit einer Baueinheit aus Filterelement 14 und Filtergehäuse 16 zweckmäßig sein.

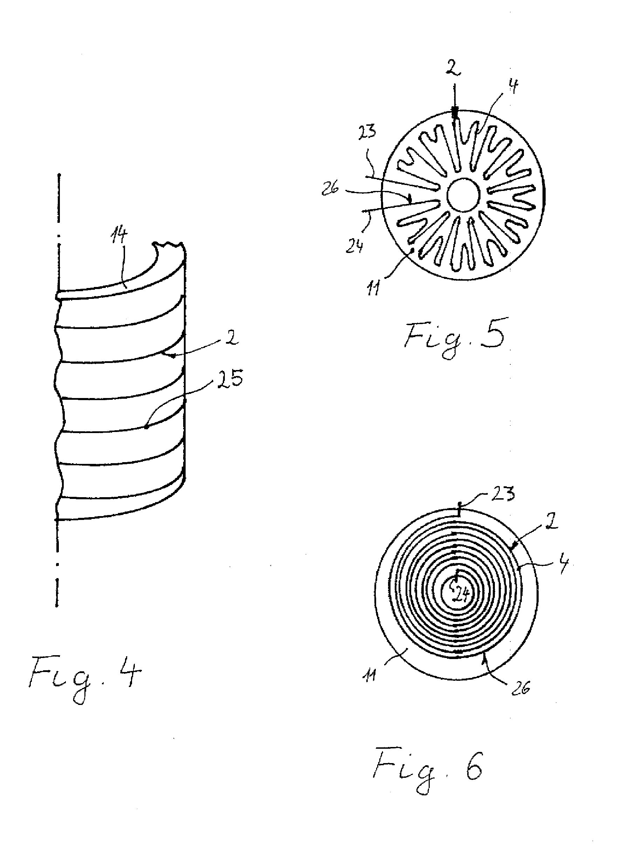

Fig. 4 zeigt in schematischer Perspektivdarstellung einen Ausschnitt aus dem Filterelement 14 nach Fig. 3. Demnach ist der Ölzustandssensor 2 als wendelförmig angeordneter Draht 25 aus Edelstahl ausgebildet und radial außenseitig auf das Filterelement 14 aufgewickelt. Es kann auch eine Verwebung, Verklebung, Vernietung oder anderweitige Festlegung des Ölzustandssensors 2 am Filterkörper 14 zweckmäßig sein, ebenso eine Festlegung auf der Innenseite.

Fig. 5 zeigt in einer Prinzipdarstellung eine Ausbildung der Endscheibe 11 nach Fig. 3, bei der der Ölzustandssensor 2 in die Endscheibe 2 integriert ist. Der als kapazitiver Sensor 4 ausgebildete Ölzustandssensor 2 verläuft oberflächenseitig der Endscheibe 11 als mäanderförmige Leiterbahn 26 und ist mit zwei radial außenliegenden Anschlußkontakten 23, 24 versehen.

Bei der in Fig. 6 gezeigten Variante der Anordnung nach Fig. 5 weist die Leiterbahn 26 zur Bildung des Sensors 2, 4 einen spiralförmigen Verlauf auf der Oberfläche der Endscheibe 11 auf, wobei je ein radial innen- bzw. außenliegender Kontakt 23, 24 zum Anschluß des Sensors 2, 4 vorgesehen ist.

## Patentansprüche

1. Ölqualitätsmeßeinrichtung für einen Ölkreislauf (1), umfassend einen Ölzustandssensor (2) und eine Auswerteelektronik (3), wobei der Ölzustandssensor (2) als kapazitiver oder impedanzspektroskopischer Sensor (4) ausgebildet und derart mit einem Filterelement (14) verbunden ist, daß bei einem Auswechseln des Filterelementes (14) zwangsweise auch der Ölzustandssensor (2) ausgewechselt wird.

2. Meßeinrichtung nach Anspruch 1,
**dadurch gekennzeichnet, daß** die Auswerteelektronik (3) an einem Filterhalter (6) des Ölfilters (5) angeordnet ist.

3. Meßeinrichtung nach Anspruch 2,
**dadurch gekennzeichnet, daß** die Auswerteelektronik, (3) in einem separaten, am Filtergehäuse (6) befestigten Gehäuse (9) angeordnet ist.

4. Meßeinrichtung nach einem der Ansprüche 1 bis 3,
**dadurch gekennzeichnet, daß** ein Filtergehäuse (16) des Filterelementes (14) in einen Filterhalter (6) einschraubbar ist, wobei für die elektrische Kontaktierung zwischen dem Ölzustandssensor (2) und der Auswerteelektronik (3) Ringkontakte (7) vorgesehen sind.

5. Meßeinrichtung nach einem der Ansprüche 1 bis 4,
**dadurch gekennzeichnet, daß** zur elektrischen Kontaktierung zwischen dem Ölzustandssensor (2) und der Auswerteelektronik (3) ein Steckverbinder (8) vorgesehen ist.

6. Meßeinrichtung nach einem der Ansprüche 1 bis 5,
**dadurch gekennzeichnet, daß** der Ölzustandssensor (2) auf der Reinölseite (10) des Ölfilters (5) angeordnet ist.

7. Meßeinrichtung nach einem der Ansprüche 1 bis 6,
**dadurch gekennzeichnet, daß** der Ölzustandssensor (2) in eine Endscheibe (11) des Ölfilters (5) integriert ist.

8. Meßeinrichtung nach Anspruch 7,
**dadurch gekennzeichnet, daß** der Ölzustandssensor (2) spiralförmig ausgebildet ist.

9. Meßeinrichtung nach Anspruch 7,
**dadurch gekennzeichnet, daß** der Ölzustandssensor (2) mäanderförmig ausgebildet ist.

10. Meßeinrichtung nach einem der Ansprüche 1 bis 6,
**dadurch gekennzeichnet, daß** der Ölfilter (5) einen jeweils etwa zylindrischen Stützkörper (12) und ein den Stützkörper (12) umschließendes Filterelement (14) umfaßt, wobei der Ölzustandssensor (2) als Hohlkörper (5) ausgebildet und zwischen dem Stützkörper (12) und dem Filterelement (14) angeordnet ist.

11. Meßeinrichtung nach einem der Ansprüche 1 bis 6,
**dadurch gekennzeichnet, daß** der Stützkörper (12) als Ölzustandssensor (2) ausgebildet ist.

12. Meßeinrichtung nach einem der Ansprüche 1 bis 6,
**dadurch gekennzeichnet, daß** der Ölzustandssensor (2) auf das Filterelement (14) fest aufgebracht ist.

13. Meßeinrichtung nach einem der Ansprüche 1 bis 12,
**dadurch gekennzeichnet, daß** der Ölzustandssensor (2) und die Auswerteelektronik (3) zur Messung mittels der Impedanzspektroskopie ausgelegt sind.

14. Meßeinrichtung nach einem der Ansprüche 1 bis 13,
**dadurch gekennzeichnet, daß** der Ölkreislauf (1) ein Schmierölkreislauf eines Verbrennungsmotors insbesondere in einem Kraftfahrzeug ist.
